# EUROPÄISCHE PATENTSCHRIFT

(11) **EP 0 695 749 B1**
(45) Veröffentlichungstag und Bekanntmachung des Hinweises auf die Patenterteilung: **25.11.1998**
(21) Anmeldenummer: 95106876.6
(22) Anmeldetag: 06.05.1995
(51) Int. Cl.: C07D 409/06, C07D 233/74, C07D 233/76, C07C 51/36, C07C 55/10

(54) **Verfahren zur selektiven Hydrierung von aktivierten, ethylenisch ungesättigten Verbindungen**
Process for the selective hydrogenation of activated, ethylenically unsaturated compounds
Procédé pour l'hydrogénation sélective de composés activés, éthyleniquement insaturés

(30) Priorität: 04.08.1994 DE 4427557
(43) Veröffentlichungstag der Anmeldung: 07.02.1996
(73) Patentinhaber: Rütgers Organics GmbH, 68305 Mannheim (DE)
(72) Erfinder: Orth, Winfried, Dr., D-67454 Hassloch (DE); Reng, Hans Georg, D-67304 Eisenberg (DE); Weiss, Wolfgang, Dr., D-68535 Neckarhausen (DE)
(74) Vertreter: Patentanwälte Zellentin & Partner

(56) Entgegenhaltungen:
- FR-A- 2 687 153
- CHEMISTRY AND INDUSTRY. CHEMISTRY AND INDUSTRY REVIEW, Nr.18, 3. Mai 1969, LETCHWORTH GB Seiten 583 - 584 A. CORBELLA ET AL. 'Synthesis of 3,4-dehydroproline'

## Beschreibung

Die Erfindung betrifft ein neues Verfahren zur selektiven Hydrierung von aktivierten, ethylenisch ungesättigten Verbindungen.

Insbesondere betrifft diese Erfindung ein Verfahren zur Hydrierung von Verbindungen mit entsprechenden Doppelbindungen an einem heterocyclischen Ring unter Erhalt eines empfindlichen, aromatischen Rings oder Ringsystems bzw. ohne üblicherweise als Katalysatorgifte wirkende Abbauprodukte, wie z.B. Schwefelverbindungen, freizusetzen.

Wirtschaftlich von Interesse sind Verbindungen mit exocyclischen Doppelbindungen als Zwischenprodukte für die Herstellung von in der Natur unbekannten Aminosäuren für die Synthese pharmazeutisch verwendbarer Peptide.

Erhalten werden solche Verbindungen durch die Kondensation von aliphatischen oder aromatischen Aldehyden mit aktivierten Methylengruppen cyclischer Verbindungen.

Als selektive Hydrierungsreagentien für entsprechende aromatische Methylenverbindungen werden in der Literatur Jodwasserstoffsäure in Eisessig in Gegenwart von rotem Phosphor beschrieben (Gattermann, Wieland, "Die Praxis des organischen Chemikers", 43. Aufl., Verl. Walter Gryter. 1982, S. 372). Diese Methoden bereiten jedoch in der technischen Anwendung aufgrund der Reaktivität der Edukte Probleme, da lange Reaktionszeiten und relativ große Mengen an J₂ oder HJ erforderlich sind, wenn eine weitgehende Umsetzung des Edukts angestrebt wird. Da lange Reaktionszeiten gleichzeitig zur vermehrten Bildung unerwünschter Hydrierungsprodukte führen, ist diese Methode als unwirtschaftlich anzusehen.

Aus Chemistry and Industry 1969 (18), 583-584 ist es bekannt, 2-Pyrrolcarboxamid mit Phosphoniumjodid (PH₄J) in rauchender Jodwasserstoffsäure zu 3,4-Dehydroprolinamid zu reduzieren. Die Herstellung von Phosphoniumjodid ist jedoch aufwendig. Dies wurde daher für die Reduktion von 2-Pyrrolcarbonsäure durch eine Mischung aus Essigsäure, H₃PO₂ und gasförmigem HJ bei -10 °C ersetzt. Nach 5-7 h Reaktion wird eine Ausbeute von 60 % d. Th. an 3.4-Dehydroprolin erhalten. Auch diese Reaktion ist sehr aufwendig.

Gemäß DE-A 43 01 588 bzw. FR 2.687.153 wird Thienylmethylidenhydantoin im Labormaßstab in einem System aus HJ, Eisessig und einem Phosphorderivat aus der durch roten Phosphor und Phosphonsäure gebildeten Gruppe mit verhältnismäßig guter Ausbeute hydriert. In den beispielhaft erwähnten Umsetzungen dient roter Phosphor bzw. phosphorige Säure als Reduktionsmittel, das jeweils in wasserfreier Form eingesetzt wird. Das zur Reaktion notwendige Wasser wird in der stöchiometrisch benötigten Menge dem Reaktionsgemisch zugegeben.

Trotz langer Reaktionszeit (bis zu 20 h) ist das Endprodukt nicht rein: - das an sich weiße (2-Thienylmethyl)-5-hydantoin kristallisiert in Form gelber Kristalle aus, die sich nur sehr schwer reinigen lassen.

Ähnliche Ergebnisse wurden in eigenen Laborversuchen bei der Hydrierung von p-Chlorphenylmethylidenhydantoin, 5-(Naphthyl-1-methyliden)-hydantoin und 5-(Pyridyl-4-methyliden)-hydantoin und anderen Hydantoinen mit exocyclischen Doppelbindungen mit rotem Phosphor in Gegenwart von Jodwasserstoffsäure erhalten, wobei die beiden letztgenannten Hydantoine dazu neigen, vermehrt unerwünschte Nebenprodukte zu bilden. Diese durch Überhydrierung entstehenden Nebenprodukte lassen sich zwar durch kürzere Reaktionszeiten zurückdrängen, jedoch wird dann keine vollständige Umsetzung der Ausgangsverbindung erzielt. Außerdem können die nach dieser Methode erhaltenen Versuchsergebnisse im technischen Maßstab nicht nachvollzogen werden.

Aufgabe der Erfindung war es daher, ein Verfahren zur Verfügung zu stellen, durch das selektiv und in hoher Reinheit möglichst in kurzen Reaktionszeiten sowohl empfindliche, zur Weiterreaktion neigende Verbindungen mit aktivierten Doppelbindungen in einfacher Weise mit hohen Ausbeuten hydriert werden können, als auch solche Verbindungen, die bei der Überreduktion Katalysatorgifte freisetzen.

Die Lösung der Aufgabe erfolgt durch ein Verfahren gemäß der Ansprüche 1 bis 7.

Es wurde gefunden, daß sich Verbindungen mit aktivierten, ethylenisch ungesättigten Gruppen durch Unterphosphorige Säure in Gegenwart von katalytischen Mengen Jod oder Jodwasserstoff und einer organischen Säure, die gleichzeitig als Lösungsmittel dient, bei kurzer Reaktionsdauer selektiv an der Doppelbindung hydrieren lassen, wenn bestimmte Reaktionsbedingungen eingehalten werden. Dies gilt insbesondere auch bei heterocyclischen Verbindungen mit exocyclischen Doppelbindungen. Überraschend dabei ist nicht die Hydrierung an sich, sondern die Tatsache, daß die Hydrierung selektiv erfolgt und die Endprodukte in hoher Reinheit erhalten werden, denn Unterphosphorige Säure ist ein wesentlich stärkeres Reduktionsmittel, als die als Stand der Technik gebrauchte Phosphorige Säure und der Fachmann würde daher eine verstärkte Bildung unerwünschter Nebenprodukte erwarten, die u.a. aus der reduktiven Spaltung heterocyclischer Ringe resultieren.

Erfindungsgemäß hydriert werden können alle Verbindungen mit aktivierten ethylenisch ungesättigten Gruppen. Aktivierte ethylenisch ungesättigte Gruppen sind ethylenisch ungesättigte Gruppen, die in unmittelbarer Nachbarschaft mindestens eine elektronegative Gruppe besitzen. Beispiele für derartige elektronegative Gruppen sind Halogenatome, Carbonyl-, Carboxyl-, Hydroxyl-, Amino-, Nitro-, Sulfonyl- oder Nitrilgruppen.

Beispiele für erfindungsgemäß hydrierbare Verbindungen sind Fumarsäure, Acrylnitril, 1-Cyclohexenamin oder Zimtsäure. Von besonderem Interesse ist die erfindungsgemäße Reduktion für heterocyclische Verbindungen mit exocyclischen Doppelbindungen. Beispiele hierfür sind
5-(Thienylmethyliden)-2,4-imidazolidindion,
5-(p-Chlorphenylmethyliden)-2,4-imidazolidindion,
5-(Naphthylmethyliden)-2,4-imidazolidindion,
5-(Pyridylmethyliden)-2,4-imidazolidindion
sowie die entsprechenden Furan-, Pyrrol-, Anthranyl- und p-Cyanophenylmethyliden-2,4-imidazolidindione und andere 2,4-Imidazolidindione mit exocyclischen Doppelbindungen. Dies ist für die genannten Cyanoverbindungen besonders überraschend, da sie selbst mit an sich schwächeren Reduktionsmitteln an dieser Bindung nicht selektiv hydriert werden können, sondern auch die p-Cyanophenylgruppe an der Nitrilfunktion hydriert wird.

Die Durchführung des Verfahrens erfolgt in der Art, daß die Ausgangsverbindung, die organische Säure und Jod oder Jodwasserstoff, letzterer bevorzugt als wäßrige Lösung, vorgelegt und das Reduktionsmittel Unterphosphorige Säure in Form einer mindestens 40 %igen, bevorzugt 50 %igen Lösung gesteuert zugegeben wird, wobei das Reaktionsgemisch so erwärmt wird, daß es unter Rückfluß siedet. Die Temperatur soll dabei über 113 °C, bevorzugt im Temperaturbereich von 115-125 °C liegen. Dies kann erreicht werden, indem die Reaktion unter Druck durchgeführt wird. In diesem Fall kann die gesamte Menge der zur Hydrierung notwendigen wäßrigen Lösung der Unterphosphorigen Säure zu Beginn der Reaktion zugegeben werden.

Aus wirtschaftlichen Gründen bevorzugt ist jedoch die Durchführung des Verfahrens bei Atmosphärendruck in einem offenen System. Dabei lassen sich Siedepunkte oberhalb 113 °C nur bei niedrigen Wassergehalten des Reaktionsgemisches erhalten. Daher wäre anzustreben, nur die stöchiometrisch notwendige Wassermenge zum Reaktionsgemisch zu geben.

Es wurde aber gefunden, daß ein Überschuß an Wasser notwendig ist, um reine Endprodukte zu erhalten.

Die Lösung dieses Problems erfolgt dadurch, daß in einem offenen System die wäßrige Lösung der Unterphosphorigen Säure gesteuert zugegeben wird. Dies kann kontinuierlich oder diskontinuierlich in mehreren Zugabeschritten erfolgen, wobei jeweils so viel der wäßrigen Lösung zum Reaktionsgemisch gegeben wird, daß eine Sumpftemperatur von 113 °C nicht unterschritten wird und die jeweils erneute Zugabe dann erfolgt, wenn reaktionsbedingt ausreichend Wasser verbraucht ist und der Siedepunkt des Reaktionsgemisches entsprechend angestiegen ist. Die Reaktionstemperatur des Gemisches schwankt daher bei der diskontinuierlichen, portionsweise Zugabe der wäßrigen Lösung im Temperaturbereich von 113 bis 130 °C, bevorzugt im Bereich von 115 bis 125 °C.

Die kontinuierliche Zugabe der wäßrigen Lösung der Unterphosphorigen Säure wird bevorzugt so gesteuert, daß die Reaktionstemperatur anfangs einigermaßen konstant im Bereich von 115 bis 120 °C und gegen Ende der Reaktion im Bereich von 120 bis 125 °C liegt.

Nach Ablauf der Reaktion werden die Endprodukte jeweils in an sich bekannter Weise vom Reaktionsgemisch abgetrennt und gereinigt. In der Regel, bei wasserunlöslichen Endprodukten, werden diese durch Wasserzugabe aus dem Reaktionsgemisch ausgefällt.

Als organische Säure, die hauptsächlich als Lösungsmittel für das Ausgangs- und Endprodukt dient wird eine möglichst wasserfreie organische Säure verwendet, die unter den Bedingungen der Reaktion inert ist und die einen pK-Wert im Bereich von 4 bis 5 hat. Beispiele sind gesättigte Carbonsäuren mit 2 bis 6 C-Atomen, aber auch Dicarbonsäuren.

Gegebenenfalls kann das Reaktionsgemisch weitere hochsiedende, inerte löslichkeitsverbessernde Mittel wie z.B. Glykol oder N-Methylpyrrolidon enthalten. Die Menge der eingesetzten Säure soll ausreichend sein, daß auch nach der Zugabe der gesamten Menge Unterphosphoriger Säure das Edukt in Lösung bleibt. Bei der Verwendung von Eisessig als Lösungsmittel sind dieses bezogen auf ein Mol Edukt etwa 300 bis 5000 ml. Die Unterphosphorige Säure wird in mindestens der gleichen molaren Menge wie das Edukt eingesetzt. Bevorzugt wird ein Überschuß an Reduktionsmittel von mindestens 10 %. Im allgemeinen werden Edukt, Unterphosphorige Säure und Jod bzw. Jodwasserstoff in molaren Verhältnissen von etwa 0,9:1:0,025 bis 1,2:5:0,05 eingesetzt.

Durch dieses Reduktionsverfahren werden nach Beendigung der Reaktion weiße, reine Produkte erhalten. Außerdem werden im Vergleich zu bisher bekannten Methoden hohe Ausbeuten erzielt. Beispielsweise betragen die Ausbeuten der Hydrierung von
5-(2-Thienylmethylen)-2,4-imidazolidindion zu
5-(2-Thienylmethyl)-2,4-imidazolidindion 90 bis 95 % der Theorie.

### BEISPIELE

### Beispiel 1

### 5-(2-Thienylmethyl)-2,4-imidazolidindion

Eine Mischung aus 300 ml Essigsäure, 97,1 g (0,5 mol) 5-(2-Thienylmethylen)-2,4-imidazolidindion, 46,2 g (0,35 mol) Unterphosphorige Säure (50 %ig), sowie 4,83 g Jod (0,019 mol) werden zwei Stunden unter Rückfluß erhitzt. Zu Beginn der Reaktion liegt der Siedepunkt des Reaktionsgemisches bei 115 °C. Er steigt innerhalb der zwei Stunden auf 119 °C. Unter Fortsetzung des Siedens werden nach zwei Stunden 46,2 g (0,35 mol) und nach einer weiteren Stunde 39,6 g 50 %ige Unterphosphorige Säure (0,3 mol) hinzugegeben. Jeweils nach Zugabe der Unterphosphorigen Säure sinkt der Siedepunkt des Reaktionsgemisches auf etwa 115 °C ab und steigt im Verlauf der Reaktion auf Werte bis 121 °C. Nach einer Nachreaktionszeit von einer Stunde wird der Einsatz mit 400 ml Wasser verdünnt und unter Rühren auf Raumtemperatur abgekühlt. Die ausgefallenen weißen Kristalle werden abgenutscht, mit Wasser gewaschen und bei 70 °C unter Vakuum getrocknet.
Schmelzpunkt: 189 - 190 °C.
Ausbeute: 89 - 92 g (90 - 93,8 % der Theorie)

### Beispiel 2

### 5-(3-Thienylmethyl)-2,4-imidazolidindion

Eine Mischung aus 300 ml Essigsäure, 97,1 g (0,5 mol), 5-(3-Thienylmethylen)-2,4-imidazolidindion, 46,2 g (0,35 mol) Unterphosphorige Säure (50 %ig), sowie 4,83 g Jod (0,019 mol) werden zwei Stunden unter Rückfluß erhitzt. Zu Beginn der Reaktion liegt der Siedepunkt des Reaktionsgemisches bei 115 °C. Er steigt innerhalb der zwei Stunden auf 119 °C. Unter Fortsetzung des Siedens werden nach zwei Stunden 46,2 g (0,35 mol) und nach einer weiteren Stunde 39,6 g Unterphosphorige Säure (0,3 mol) hinzugegeben. Jeweils nach Zugabe der Unterphosphorigen Säure sinkt der Siedepunkt des Reaktionsgemisches auf etwa 115 °C ab und steigt im Verlauf der Reaktion auf Werte bis 121 °C. Nach einer Nachreaktionszeit von einer Stunde wird der Einsatz mit 400 ml Wasser verdünnt und unter Rühren auf Raumtemperatur abgekühlt. Die ausgefallenen weißen Kristalle werden abgenutscht, mit Wasser säurefrei gewaschen und bei 70 °C unter Vakuum getrocknet.
Weiße Kristalle
Schmelzpunkt: 199 - 201 °C
Ausbeute: 93,2 g (95 % der Theorie)

### Beispiel 3

### 5-(1-Naphthylmethyl)2,4-imidazolidindion

Eine Mischung aus 470 ml Essigsäure, 23,83 g 5-(l-Naphthylmethylen)-2,4-imidazolidindion (0,1 mol), 26,4 g Unterphosphorige Säure (50 %ig), sowie 0,9 g Jod werden 25 Stunden unter Rückfluß erhitzt. Nach dem Verdünnen mit 500 ml Wasser wird der Ansatz unter Rühren auf Raumtemperatur abgekühlt, wobei das Produkt kristallin ausfällt. Es wird abgenutscht, mit Wasser säurefrei gewaschen und unter Vakuum bei 70 °C getrocknet.
Farblose Kristalle
Schmelzpunkt: 260 bis 262 °C
Ausbeute: 17,9 g (74, 6 % der Theorie)

### Beispiel 4

### 5-(2-Naphthylmethyl)-2,4-imidazolidindion

Eine Mischung aus 470 ml Essigsäure, 23,83 g 5-(2-Naphthylmethylen)-2,4-imidazolidindion (0,1 mol), 26,4 g Unterphosphorige Säure (50 %ig), sowie 0,9 g Jod werden 25 Stunden unter Rückfluß erhitzt. Nach dem Verdünnen mit 500 ml Wasser wird der Einsatz unter Rühren auf Raumtemperatur abgekühlt, wobei das Produkt kristallin ausfällt. Es wird abgenutscht, mit Wasser säurefrei gewaschen und unter Vakuum bei 70 °C getrocknet.
Farblose Kristalle
Schmelzpunkt: 202 - 204 °C
Ausbeute: 19,3 g (80,4 % der Theorie)

### Beispiel 5

### Ethan-1,2-dicarbonsäure (Bernsteinsäure)

Eine Mischung aus 300 ml Essigsäure, 116 g (1 mol) Fumarsäure, 145 g (1,1 mol) unterphosphorige Säure sowie 9,65 g (ca. 0,038 mol) Jod wird 10 Stunden zum Rückfluß erhitzt. Anschließend wird ein Teil der wässrigen Essigsäure bei 15 mbar abdestilliert. Der Rückstand wird mit 200 ml Wasser verrührt und schließlich über Nacht auf 5-8 °C gekühlt. Das Kristallisat wird abgesaugt, mit Eiswasser gewaschen und im Vakuum getrocknet.
Farblose Kristalle
Schmelzpunkt 184-5 °C
Ausbeute: 97,1 (82,3 % der Theorie)

### Beispiel 6

### 5-(4-Chlorbenzyl)-2,4-imidazolidindion

Eine Mischung aus 300 ml Essigsäure, 44,5 g (0,2 mol) 5-(4-Chlorphenylmethyliden)-2,4-imidazolidindion, 18,5 g (0,14 mol) unterphosphorige Säure, 50-proz., sowie 1,93 g (78 mmol) Jod wird 3 Stunden unter Rückfluß erhitzt. Dann werden unter Rückfluß weitere 18,5 g (0,14 mol) unterphosphorige Säure und nach zwei zusätzlichen Stunden nochmals 15,8 g (0,12 mol) unterphosphorige Säure hinzugefügt. Nachdem die Mischung nach drei weiteren Stunden unter Rückfluß erhitzt wurde, wird die Mischung mit 500 ml Wasser versetzt, kurz aufgekocht und schließlich unter Rühren auf Raumtemperatur abgekühlt. Das Kristallisat wird abgesaugt, mit Eiswasser säurefrei gewaschen und getrocknet.
Weißer Feststoff vom Schmelzpunkt: 240 °C
Ausbeute: 39,1 g (87 % der Theorie).

## Patentansprüche

1. Verfahren zur selektiven Hydrierung von Verbindungen mit ethylenisch ungesättigten Gruppen, welche in unmittelbarer Nachbarschaft eine aktivierende elektronegative Gruppe besitzen, in Gegenwart einer organischen Säure, Wasser und einer katalytischen Menge Jod oder Jodwasserstoff, **dadurch gekennzeichnet**, daß durch Zugabe einer mindestens 40 %igen wässrigen Lösung von Unterphosphoriger Säure reduziert wird, wobei die Zugabe der Lösung der Unterphosphorigen Säure und äußere Wärmezuführung so gesteuert werden, daß die Temperatur des unter Rückfluß siedenden, wasserhaltigen Reaktionsgemisches über 113 °C liegt.

2. Verfahren nach Anspruch 1, **dadurch gekennzeichnet**, daß die Unterphosphorige Säure in Form einer 50 %igen wäßrigen Lösung eingesetzt wird.

3. Verfahren gemäß den Ansprüchen 1 und 2, **dadurch gekennzeichnet,** daß das Verfahren in einem offenen System durchgeführt wird und die wäßrige Lösung der Unterphosphorigen Säure portionsweise zugegeben wird.

4. Verfahren gemäß den Ansprüchen 1 bis 3, **dadurch gekennzeichnet**, daß als organische Säure eine Säure aus der Gruppe der gesättigten, bei Raumtemperatur flüssigen Kohlenwasserstoffsäuren mit einem pK-Wert von etwa 4,5 bis 5 verwendet wird.

5. Verfahren gemäß Anspruch 4, **dadurch gekennzeichnet**, daß als organische Säure eine Säure aus der Gruppe Essigsäure, Propionsäure oder Glykolsäure verwendet wird.

6. Verfahren gemäß den Ansprüchen 1 bis 5, **dadurch gekennzeichnet**, daß die zu hydrierende Verbindung, die Unterphosphorige Säure und Jod in molaren Verhältnissen von 0,9:1:0,025 bis 1,2:5:0,05 eingesetzt werden.

7. Verfahren gemäß den Ansprüchen 1 bis 6, **dadurch gekennzeichnet**, daß die zu hydrierende Verbindung, die Unterphosphorige Säure und Jod in einem molaren Verhältnis von 1:2:0,038 eingesetzt werden.

## Claims

1. Process for the selective hydrogenation of compounds with ethylenically-unsaturated groups which, in the immediate neighbourhood, possess an activating electronegative group, in the presence of an organic acid, water and of a catalytic amount of iodine or hydrogen iodide, characterised in that it is reduced by addition of an at least 40% aqueous solution of hypophosphorous acid, whereby the addition of the solution of the hypophosphorous acid and external heat supply are so controlled that the temperature of the water-containing reaction mixture boiling under reflux lies above 113°C.

2. Process according to claim 1, characterised in that the hypophosphorous acid is used in the form of a 50% aqueous solution.

3. Process according to claims 1 and 2, characterised in that the process is carried out in an open system and the aqueous solution of the hypophosphorous acid is added portionwise.

4. Process according to claims 1 to 3, characterised in that, as organic acid, there is used an acid of the group of the saturated hydrocarbon acids liquid at room temperature with a pK value of about 4.5 to 5.

5. Process according to claim 4, characterised in that, as organic acid, there is used an acid of the group acetic acid, propionic acid or glycolic acid.

6. Process according to claims 1 to 5, characterised in that the compound to be hydrogenated, the hypophosphorous acid and iodine are used in molar ratios of 0.9:1.0:0.025 to 1.2:5:0.05.

7. Process according to claims 1 to 6, characterised in that the compound to be hydrogenated, the hypophosphorous acid and iodide are used in a molar ratio of 1:2:0.038.

## Revendications

1. Procédé pour l'hydrogénation sélective de composés contenant des groupes éthyléniquement insaturés qui possèdent, à proximité immédiate, un groupe d'activation électronégatif, en présence d'un acide organique, d'eau et d'une quantité catalytique d'iode ou d'acide iodhydrique, caractérisé en ce qu'on procède à une réduction par addition d'acide sous-phosphorique sous forme d'une solution aqueuse à raison d'au moins 40%, dans lequel on règle l'addition de la solution d'acide sous-phosphorique et l'apport de chaleur externe de telle sorte que la température du mélange de réaction aqueux en ébullition sous reflux est supérieure à 113°C.

2. Procédé selon la revendication 1, caractérisé en ce qu'on met en oeuvre l'acide sous-phosphorique sous la forme d'une solution aqueuse à 50%.

3. Procédé selon les revendications 1 et 2, caractérisé en ce qu'on effectue le procédé dans un système ouvert et on ajoute la solution aqueuse de l'acide sous-phosphorique, par portions.

4. Procédé selon les revendications 1 à 3, caractérisé en ce qu'on utilise, à titre d'acide organique, un acide choisi parmi le groupe constitué par des acides d'hydrocarbures saturés liquides à la température ambiante, possédant une valeur de pK d'environ 4,5 à 5.

5. Procédé selon la revendication 4, caractérisé en ce qu'on utilise, à titre d'acide organique, un acide choisi parmi le groupe constitué par l'acide acétique, l'acide propionique ou l'acide glycolique.

6. Procédé selon les revendications 1 à 5, caractérisé en ce qu'on met en oeuvre le composé à hydrogéner, l'acide sous-phosphorique et l'iode, dans des rapports molaires de 0,9:1:0,025 à 1,2:5:0,05.

7. Procédé selon les revendications 1 à 6, caractérisé en ce qu'on met en oeuvre le composé à hydrogéner, l'acide sous-phosphorique et l'iode dans un rapport molaire de 1:2:0,038.
